# EUROPEAN PATENT APPLICATION

(11) **EP 4 410 279 A1**
(43) Date of publication of application: **07.08.2024**
(21) Application number: 24153799.2
(22) Date of filing: 24.01.2024
(51) Int. Cl.: A61K 9/20, A61K 31/426

(54) **A FILM TABLET COMPRISING MIRABEGRON**

(30) Priority: 25.01.2023 TR 202300865
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); SEZER, Dilay, Istanbul (TR); KARABULUT, Serif, Istanbul (TR)
(74) Representative: Sevinç, Erkan

(57) **Abstract**

The present invention relates to a film coated tablet comprising mirabegron, polyethylene oxide and at least one pharmaceutically acceptable excipient wherein the amount of polyethylene oxide is 55.0% to 75.0% by weight in the total composition. The present invention also relates to a simple, rapid, cost effective, time-saving and industrially convenient method of preparing the film coated tablet.

## Description

### Field of the Invention

The present invention relates to a film coated tablet comprising mirabegron, polyethylene oxide and at least one pharmaceutically acceptable excipient wherein the amount of polyethylene oxide is 55.0% to 75.0% by weight in the total composition. The present invention also relates to a simple, rapid, cost effective, time-saving and industrially convenient method of preparing the film coated tablet.

### Background of the Invention

Overactive bladder causes a frequent and sudden urge to urinate that may be difficult to control. You may feel like you need to pass urine many times during the day and night, and may also experience unintentional loss of urine.

There are many options for treatment of overactive bladder such as; bladder training and drug therapy using anticholinergic substances such as propiverine hydrochloride and oxybutynin hydrochloride have been mostly used at present. However, intractable cases and side effects could be occurred such as urinary dysfunction and dry mouth and, therefore, Mirabegron has been reported as one of the substance for the management of overactive bladder.

Mirabegron is a beta-3 adrenergic agonist that is used for treatment of overactive bladder syndrome. The chemical designation of mirabegron is 2-(2-amino-1,3-thiazol-4-yl)-N-[4-[2-[[(2R)-2-hydroxy-2-phenylethyl]amino]ethyl]phenyl] acetamide, with the chemical structure illustrated below in Formula I.

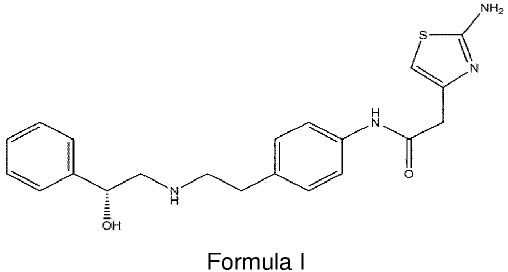

A mirabegron containing pharmaceutical product is approved under the brand name Betmiga^{®} in the EU and Mirbetriq^{®} in the US as modified release tablets comprising 25 and 50 mg of mirabegron.

Mirabegron is considered to be a Class III compound according to the Biopharmaceutical Classification System (BCS). That means that it has high solubility and low permeability. It is known that the bioavailability of mirabegron is affected by the presence of food in the GI tract. To prevent this food effect, modified release form is used.

EP1559427 patent application was first disclosed a pharmaceutical composition comprising mirabegron that can be used as a therapeutic agent for overactive bladder, such as overactive bladder accompanied by prostatic hyperplasia, or overactive bladder accompanied by urinary urgency, urinary incontinence, and urinary frequency.

WO2019072404 A1 relates to a pharmaceutical composition for modified release comprising; mirabegron presenting 5-25 wt% to the total weight of the uncoated tablet, polyethylene oxide having an average molecular weight of approximately 7,000,000 or a viscosity of 7500 to 10000 cps at a 1% aqueous solution at 25°C and polyethylene glycol having an average molecular weight of approximately 6000 to 10000, preferably 8000, wherein the weight ratio polyethylene oxide to polyethylene glycol ranges from 1:3 to 1:4.5.

WO2018169325 relates to a controlled release pharmaceutical composition comprising mirabegron or a pharmaceutically acceptable salt thereof and a polyethylene oxide as a sustained release agent.

In prior art, there are also several patents which disclose a composition comprising mirabegron. However, despite the dissolution profile and stability problems of compositions comprising mirabegron, an effective formulation and method has not been disclosed.

There still remains a need in the art to provide an improved film coating tablet formulation of mirabegron, having high solubility, dissolution rate, and excellent physicochemical properties such as flowability, compressibility and homogeneity.

### Detailed Description of the Invention

The main object of the present invention is to provide a film coating tablet comprising mirabegron with having desired level of dissolution rate which overcomes the above described problems in prior art and have additive advantages over them.

Another object of the present invention is to provide a film coating tablet comprising mirabegron with improved homogeneity, flowability and high stability.

Another object of the present invention is to provide a film coating tablet comprising mirabegron is prepared by simple, easy, time-saving and fast manufacturing methods.

The term "modified release" refers to any pharmaceutical formulation that maintain constant levels of a drug in the patient's bloodstream by releasing the drug over an extended period of time. Modified release is formulated to release the active ingredient gradually and predictably over a 12-hour to 24-hour period. In this invention, using release controlling agent (polyethylene oxide) provides modified release.

According to an embodiment of the present invention, polyethylene oxide is the release controlling agent. Polyethylene oxide is a very hydrophilic polymer. It is available in several different grades that vary in viscosity profiles in aqueous isopropyl alcohol solutions.

According to an embodiment of the present invention, a film coated tablet comprising mirabegron, polyethylene oxide and at least one pharmaceutically acceptable excipient wherein the amount of polyethylene oxide is 55.0% to 75.0% by weight in the total composition. The tablet has modified release.

According to an embodiment of the present invention, the amount of polyethylene oxide is 55.0% to 75.0% by weight in the total composition. The amount is important because the rapid or extra slow release of mirabegron can cause some problems, for example; dissolution profile and stability and half-life of the drug. By using a high percentage of PEO (55.0-75.0%), both the desired dissolution profile and stability were achieved. In addition, this high rate of use had a positive effect on powder flowability.

According to an embodiment of the present invention, the amount of polyethylene oxide is 62.0% to 70.0% by weight in the total composition.

Polyethylene oxide suitable for use in the present invention is commercially available. Polyethylene oxide WSR 205 is a non-ionic, water-soluble polyethylene oxide) polymer with a molecular weight of 600,000 with viscosity 4,500 to 8,800 cP. Polyethylene oxide WSR 205 is a water insoluble hydrophilic excipient within the tablet core has shown good results in the achievement of the desired dissolution profile.

According to an embodiment of the present invention, the release controlling agent is polyethylene oxide WSR 205. Thus, the desired release profile is achieved simply and easily using only a release agent.

According to an embodiment of the present invention, the amount of polyethylene oxide WSR 205 is 55.0% to 75.0% by weight in the total composition.

According to an embodiment of the present invention, the film coated tablet comprising mirabegron, polyethylene oxide and at least one pharmaceutically acceptable excipient wherein the weight ratio of polyethylene oxide to mirabegron ranges from 1:1 to 4:1. This specific weight ratio has shown good results in the achievement of the desired dissolution profile and compliance to the dissolution specification during the stability testing.

According to an embodiment of the present invention, the amount of mirabegron is 25.0% to 35.0% by weight in the total composition. Preferably, it is between 26.0% to 30.0% by weight in the total formulation.

According to an embodiment of the present invention, the film coating tablet comprises;
- 25.0% and 35.0% by weight of Mirabegron
- 55.0% and 75.0% by weight of Polyethylene oxide WSR 205

In general terms, excipients provided in a composition may positively or negatively influence the physicochemical and pharmacokinetic properties, e.g. the solubility, stability, absorption, bioavailability of an active agent. For this reason, the excipients which accompany an active agent have to be selected in a careful and conscious manner while a composition is developed. If active agent is incompatible of excipients, stability and psychochemical problems may be during or after the process. The tablet should have no physicochemical incompatibility between the active agent and the excipients.

According to one embodiment of the present invention, the film coated tablet comprises at least one pharmaceutically acceptable excipient which is selected from the group comprising diluents, antioxidants, lubricants or mixtures thereof.

Suitable diluents are selected from the group comprising microcrystalline cellulose, lactose monohydrate, ammonium alginate, calcium carbonate, calcium phosphate, calcium phosphate dehydrate, neutral pellets, calcium sulfate, cellulose, cellulose acetate, ethylcellulose, lactose, mannitol, magnesium carbonate, medium chain triglycerides, polyvinylpyrrolidone, sodium alginate, sodium chloride, sucrose, sugar spheres, or mixtures thereof.

According to an embodiment of the present invention, the diluent is microcrystalline cellulose. It gives volume to tablet and enables mirabegron to be dispersed homogeneously in the tablet.

According to an embodiment of the present invention, the amount of microcrystalline cellulose is 1.0% to 10.0% by weight in the total composition.

Suitable antioxidants are selected from the group comprising BHT (butylated hydroxy toluene), propyl gallate, BHA (butylated hydroxy anisole), monothioglycerol or mixtures thereof.

According to an embodiment of the present invention, the antioxidant is BHT (butylated hydroxy toluene). It helps to provide stability of tablet.

According to an embodiment of the present invention, the amount of butylated hydroxytoluene is 0.05% to 1.0% by weight in the total composition.

Suitable lubricant is selected from the group comprising magnesium stearate, polyoxyl 40 stearate, calcium stearate, sodium stearyl fumarate, potassium stearate, stearic acid, high melting point waxes, sodium chloride, sodium benzoate, sodium acetate, sodium oleate or mixtures thereof.

According to an embodiment of the present invention, the lubricant is magnesium stearate. It enhances product flow by reducing inter particulate friction.

According to an embodiment of the present invention, the amount of magnesium stearate is 0.05% to 1.5% by weight in the total composition.

According to an embodiment of the present invention, the film coating tablet comprises;
- Mirabegron
- Polyethylene oxide WSR 205
- Microcrystalline cellulose pH102
- Butylated Hydroxytoluene
- Magnesium stearate.

Furthermore, the film coating tablet is obtained by using dry granulation or direct compression and therefore a simple and low-cost production method was employed.

According to an embodiment of the present invention, a process for the preparation of the film coated tablet comprising mirabegron comprising the following steps:
- Sieving mirabegron, microcrystalline cellulose and polyethylene oxide, and adding butylated hydroxytoluene and mixing all,
- Adding the half of magnesium stearate and then mixing,
- Compressing the mixture and sieving the mixture,
- Adding the remaining of magnesium stearate and then mixing,
- Compressing the mixture into the tablet,
- Coating the tablets with film coating agent.

### Example 1:

| **Ingredients** | **Amount (% by weight of the total formulation)** |
|---|---|
| Mirabegron | 25.0 - 35.0 |
| Polyethylene oxide WSR 205 | 55.0 - 75.0 |
| Microcrystalline cellulose pH102 | 1.0 - 10.0 |
| Butylated Hydroxytoluene | 0.05 - 1.0 |
| Magnesium stearate | 0.05 - 1.5 |
| **TOTAL** | 100 |
| **Film Coating** | |

### Example 2:

| **Ingredients** | **Amount (% by weight of the total formulation)** |
|---|---|
| Mirabegron | 28.57 |
| Polyethylene oxide WSR 205 | 67.60 |
| Microcrystalline cellulose pH102 | 3.00 |
| Butylated Hydroxytoluene | 0.23 |
| Magnesium stearate | 0.60 |
| **TOTAL** | **100** |
| **Film Coating** | |

A process for example 1 or 2 ;
- Sieving mirabegron, microcrystalline cellulose and polyethylene oxide into 1 mm, adding butylated hydroxytoluene and mixing all,
- Adding the half of magnesium stearate and then mixing,
- Compressing the mixture and sieving the mixture into 1.0 mm,
- Adding the remaining of magnesium stearate and then mixing,
- Compressing the mixture into the tablet,
- Coating the tablets with film coating agent.

### Example 3: Film coating

| **Ingredients** | **Amount (% by weight of the total coating)** |
|---|---|
| HPMC | 69.50 |
| IRON OXIDE YELLOW | 17.45 |
| Glycerin | 13.05 |
| **Total** | **100** |

## Claims

1. A film coated tablet comprising mirabegron, polyethylene oxide and at least one pharmaceutically acceptable excipient wherein the amount of polyethylene oxide is 55.0% to 75.0% by weight in the total composition.

2. The film coated tablet according to claim 1, wherein the amount of polyethylene oxide is 62.0% to 70.0% by weight in the total composition.

3. The film coated tablet according to claim 1, wherein the release controlling agent is polyethylene oxide WSR 205.

4. The film coated tablet according to claim 1, wherein the weight ratio of polyethylene oxide to mirabegron ranges from 1:1 to 4:1.

5. The film coated tablet according to claim 1, wherein the amount of mirabegron is 25.0% to 35.0% by weight in the total composition.

6. The film coated tablet according to claim 1, wherein the tablet comprising;
- 25.0% and 35.0% by weight of Mirabegron
- 55.0% and 75.0% by weight of Polyethylene oxide WSR 205

7. The film coated tablet according to claim 1, wherein the film coated tablet comprises at least one pharmaceutically acceptable excipient which is selected from the group comprising diluents, antioxidants, lubricants or mixtures thereof.

8. The film coated tablet according to claim 7, wherein diluents are selected from the group comprising microcrystalline cellulose, lactose monohydrate, ammonium alginate, calcium carbonate, calcium phosphate, calcium phosphate dehydrate, neutral pellets, calcium sulfate, cellulose, cellulose acetate, ethylcellulose, lactose, mannitol, magnesium carbonate, medium chain triglycerides, polyvinylpyrrolidone, sodium alginate, sodium chloride, sucrose, sugar spheres, or mixtures thereof.

9. The film coated tablet according to claim 7, wherein the diluent is microcrystalline cellulose.

10. The film coated tablet according to claim 9, wherein the amount of microcrystalline cellulose is 1.0% to 10.0% by weight in the total composition.

11. The film coated tablet according to claim 1, wherein the tablet comprising;
- Mirabegron
- Polyethylene oxide WSR 205
- Microcrystalline cellulose pH102
- Butylated Hydroxytoluene
- Magnesium stearate.

12. A process for the preparation of the film coated tablet comprising mirabegron comprising the following steps:
- Sieving mirabegron, microcrystalline cellulose and polyethylene oxide, and adding butylated hydroxytoluene and mixing all,
- Adding the half of magnesium stearate and then mixing,
- Compressing the mixture and sieving the mixture,
- Adding the remaining of magnesium stearate and then mixing,
- Compressing the mixture into the tablet,
- Coating the tablets with film coating agent.
